# EUROPEAN PATENT APPLICATION

(11) **EP 4 800 021 A1**
(43) Date of publication of application: **02.09.2026**
(21) Application number: 25160196.9
(22) Date of filing: 26.02.2025
(51) Int. Cl.: C07K 1/02, C07K 1/04, C07K 1/06

(54) **DIETHYLENETRIAMINE AS BASE IN PEPTIDE SYNTHESIS**

(71) Applicant: VITO NV, 2400 Mol (BE); Alma Mater Studiorum - Università di Bologna, 40126 Bologna (IT)
(72) Inventor: ORMEROD, Dominic John, 2400 Mol (BE); MONAGHEDDU, Marzio, 2400 Mol (BE); CABRI, Walter, 40126 Bologna (IT); TOLOMELLI, Alessandra, 40126 Bologna (IT); FERRAZZANO, Lucia, 40126 Bologna (IT); CORBISIERO, Dario, 40126 Bologna (IT)
(74) Representative: Arnold & Siedsma

(57) **Abstract**

The invention relates to an improvement on the removal of the Fmoc N-terminal protecting group in the synthesis of peptides; in particular in the synthesis of peptides using Solid Phase Peptide Synthesis (SPPS) or using discriminating membrane technology to separate out byproducts and excess reagents from the reaction mixture; more in particular in the synthesis of peptides using discriminating membrane technology to separate out byproducts and excess reagents from the reaction mixture.

## Description

### Technical field

The present invention is related to an improved method for peptide manufacture, making use of reactor with discriminating membrane technology to separate out byproducts and excess reagents from the reaction mixture using green organic solvents.

The discriminating membrane technology is based on the application of a membrane which is configured to act as a solid phase extraction membrane between a reaction chamber and an extraction chamber present within the reactor with an affinity for reagents and by-products present in the reaction chamber thereby limiting the amount of organic solvents and water necessary to complete the coupling-deprotection sequence that need to be iterated.

The invention relates to an improvement on the removal of the Fmoc N-terminal protecting group in the synthesis of peptides; in particular in the synthesis of peptides using Solid Phase Peptide Synthesis (SPPS), Liquid Phase Peptide Synthesis (LPPS) or using discriminating membrane technology to separate out byproducts and excess reagents from the reaction mixture; more in particular in the synthesis of peptides using discriminating membrane technology to separate out byproducts and excess reagents from the reaction mixture.

### Background art

In peptide chemical synthesis membrane-based technologies are iterative technologies like Solid Phase Peptide Synthesis (SPPS) or Liquid Phase Peptide Synthesis (LPPS) (Green Chemistry 2022, 24, 975-1020). The membrane-based technology can be applied to fluorenylmethoxycarbonyl (Fmoc) LPPS for peptides, which is the most commonly used method, wherein the Fmoc group is used as the amino protective group in the stepwise peptide synthesis approach.

The use of a separation barrier between a reaction chamber and supply chamber to separate out the byproducts and excess reagents is known in the art of peptide synthesis via LPPS. In those solutions the membrane acts simply as a physical barrier with a molecular cut-off value to retain the reaction product in the reaction chamber.

The barriers used in such systems may be provided in a variety of ways, such as porous membranes, porous particles, hollow fibers, fritted discs, or the like. The porous barrier may be a single or multiple layer, may be in the form of sheets, particles, hollow fibers, tubes, or the like. Numerous configurations may be designed which allow for maintenance of the desired components in the reaction region with the removal of the undesired components from the reaction region. By providing for a source of the low molecular weight reactants to the reaction region, so as to replenish or augment the depletion of such components during the course of the reaction, while at the same time removing the low molecular weight products of the reaction, one can greatly extend the course of the period of expression and provide for high molar ratios of protein or oligonucleotide product to template present in the reaction medium.

In its simplest configuration these systems consist of a membrane bag containing the reaction region, while retaining a solution outside the membrane which provides for the desired level of the lower molecular weight reaction components in the reaction region. Thus, by exchange across the membrane, the lower molecular weight products produced by the reaction will be continuously dialyzed into the external solution, while the reaction components will be continuously replenished in the reaction region. A down-size of these systems applied to peptides and oligonucleotide synthesis is that large volumes of the external solution are required to keep a proper flux across the barrier and keep the amount of low molecular weight products within the reaction region to a minimum; The process mass intensity (PMI) that is the total process mass kg/kg of product is dramatically increasing with respect to the solid phase technology, increasing also the process costs.

Another drawback is the increasing of process time. Again in comparison to solid phase technology, the length of time required to perform this operation is longer than that required to wash impurities away from a growing peptide anchored onto a solid phase resin. This is principally due to the fact that the volume of solvent required to wash impurities out of a system using the membrane process of Livingston is greater much than that required in solid phase synthesis and as such unless prohibitively large membrane surface area's are used, the operation time will be quite long.

In co-pending PCT application PCT/EP2024/074574 such an improved system for peptide synthesis, schematically represented in Figure 2, has been provided wherein a solid phase extraction membrane is positioned between the reaction chamber and extraction chamber, wherein said solid phase extraction membrane has a surface polarity that matches the polarity of the reaction solution, with an affinity for reagents and optionally also for by-products present in the reaction chamber, and wherein said solid phase extraction membrane has a surface polarity contrary to the polarity of the extraction solution.

However, this system is still faced with the separation challenge in achieving an optimal removal of the reagents and by-products present in the reaction chamber.

Thus, it would be desirable to develop an improved system for peptide synthesis that can operated at a low trans-membrane pressure and that produces less unwanted reaction ballast in the reaction chamber that can affect downstream processing.

### Summary of invention

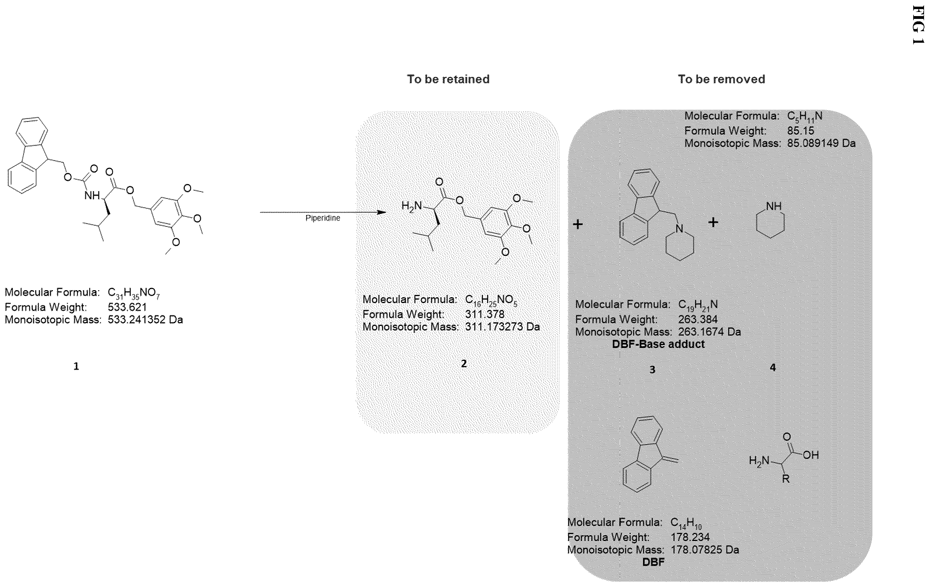

In current peptide synthesis, the Fmoc N-terminal protecting group is removed after each amide bond formation cycle in the stepwise solid state or liquid phase peptide synthesis method, under basic conditions typically in the presence of an organic amine, such as for example but not limited to piperidine, pyrrolidine, diethyl amine, and other secondary amine as well as primary amines like tBuNH2 or dimethyl amino propyl amine (DEAPA). Secondary amine like piperidine most often being used; typically, by contacting the growing peptide with a solution of 20*%*wt piperidine in DMF, dibenzofulvene (DBF) that is generated during Fmoc deprotection is trapped by the base in a DBF-Base adduct (see Figure 1).

Per reference to Tables 2 in the examples hereinafter, it has been observed that not all bases will be successful in trapping the DBF and prevent double coupling given the excess of amino acid being used in each step of the peptide synthesis.

In the case of tBuNH2 or DEAPA the deprotection is not followed by DBF trapping because of the lower basicity of the primary amines. The development of a process where the formation of the peptide bond is immediately followed by base treatment eliminating the washing steps is critical to contain solvents consumption and speed up the reaction. In this context it is critical to have a non hindered primary amine that is reacting with the excess of activated amino acid and subsequently perform Fmoc deprotection and DBF trapping. If the sequence of events it is not correct the risk of double coupling is extremely high. Piperidine is deprotecting very rapidly the growing peptide with the formation of the DBF-base adduct but without quenching the activated amino acid favoring the double coupling product. tBuNH2 is too hindered to react with the activated amino acid and the formation of double coupling is observed, in addition it is not reacting with DBF to from the DBF-adduct DEAPA has a primary amine that can react with the excess of acylating reagent but the deprotection is less efficient than piperidine since again it is not generating the DBF adduct. It has now been discovered that using DETA (diethylenetriamine; CAS N° - 110-40-0) as the base reagent we have the combination of two primary amines that react with the excess of the activated amino acid, while the secondary amine is promoting Fmoc deprotection trapping DBF resulting in a highly efficient removal of the DBF-base adduct from the reaction environment. The procedure allows to decrease the use of solvents used for the washings in SPPS, allowed to get rapid removal after coupling-deprotection of all the side products that have plenty of salified amino groups belonging to the DEAT fragment. The use of DETA represent an improvement respect to the base used so far in the prior art for Fmoc removal.

It is accordingly an objective of the present invention to provide the use of DETA as the base reagent in the synthesis of peptides. In an embodiment, the present invention provides the use of DETA as base reagent in the synthesis of peptides by means of membrane-based technologies like Solid Phase Peptide Synthesis (SPPS) or Liquid Phase Peptide Synthesis (LPPS). In a particular embodiment, the present invention provides the use of DETA as base reagent in the synthesis of peptides by means of Liquid Phase Peptide Synthesis (LPPS).

Further, the present invention provides a method for the cleavage of the Fmoc amino acid protective group by using a solution comprising DETA in the synthesis of peptides; in particular in the synthesis of peptides by means of membrane-based technologies like Solid Phase Peptide Synthesis (SPPS) or Liquid Phase Peptide Synthesis (LPPS); more in particular in the synthesis of peptides by means of Liquid Phase Peptide Synthesis (LPPS).

In an embodiment of the present invention, the method comprises a step of contacting the Fmoc protected amino acid groups with a solution comprising DETA, thereby cleaving the Fmoc from said Fmoc protected amino acid groups.

In the methods according to the invention, the solution comprising DETA is preferably a solution of DETA in the reaction solvent being used. For example, in case of SPPS, the reaction solvent is typically a polar aprotic solvent, in particular selected from ethyl acetate, anisole or DMF. When used in the synthesis of peptides of peptides by means of Liquid Phase Peptide Synthesis (LPPS), best results were obtained using a solution of DETA in anisole as reaction solution. Compared to the use of piperidine as base reagent in the same reaction solution, DETA forms the dibenzofulvene (DBF)-base adduct more efficiently than piperidine, with less than 5% of free dibenzofulvene (DBF) remaining in the reaction mixture after deprotection, compared to up to 15*%* of free DBF when piperidine is used as base reagent in the Fmoc amino deprotection step.

Hence, in a preferred embodiment the solution of DETA used in the methods according to the invention, is a solution of DETA in anisole; in particular a solution from about 5*%* to 30 Vol *%* of DETA in anisole, in particular a solution of 10 Vol*%* of DETA in anisole.

In an embodiment, the method provides the use of DETA as base reagent in the synthesis of peptides by means of a liquid phase peptide synthesis system having a housing (1) comprising a reaction solution (7) in a reaction chamber (2) and an extraction solution (8) in an extraction chamber (3) separated from each other by a membrane (4), characterized in that the membrane is configured to act as a solid phase extraction membrane between the reaction chamber and extraction chamber with an affinity for reagents and by-products present in the reaction chamber.

In an embodiment of the aforementioned method, the use of DETA as a base reagent comprises a step of feeding a solution of DETA into the reaction chamber thereby contacting the Fmoc protected amino acid groups present within said reaction chamber with DETA, and effecting removal of Fmoc from said Fmoc protected amino acid groups. In a particular embodiment Fmoc is removed from the amino groups with the formation of a BDF-base adduct. Using a solution of DETA in anisole the removal of the Fmoc through the formation of the BDF-base adduct is quasi complete with less than 5*%* of free dibenzofulvene (DBF) remaining in the reaction mixture after deprotection.

In addition, the DBF-base adduct obtained with DETA as base reagent is completely removed from the reaction chamber through the solid phase extraction membranes used in the liquid phase peptide synthesis system herein disclosed. Removal of said adduct from the reaction chamber into the extraction chamber can be effected after every single step of the amino acid coupling-deprotection sequence in the stepwise synthesis of the peptides .

In an embodiment of the invention, the membranes are polymeric or ceramic nanofiltration membranes, in particular polymeric membranes with a Molecular Weight Cut-Off (MWCO) of less than 900.

The most common ceramic membranes are made of Al, Si, Ti or Zr oxides, with Ti and Si being more stable than Al or Si oxides. In some less frequent cases, Sn or Hf are used as base elements. Each oxide has a different surface charge in solution, with different surface characteristics in sense of surface polarity. Other membranes can be composed of mixed oxides of two of the previous elements, or are established by some additional compounds present in minor concentration.

In a particular embodiment the membrane is polymeric membrane selected from a silicone - coated polyacrylonitrile or a polystyrene modified hymem mebrane, the silicone - coated polyacrylonitrile being most preferred, such as the commercially available PURAMEM ^{©} Selective of Evonik Operations GmbH. It has been observed that the combination of this type of low polarity membrane(s) with anisole as reaction solution and DETA as base reagent provides the best results in LPPS .

In an embodiment the LPPS system as provided in [00018], further comprises a feed section (5) and an extraction section (6) wherein the feed section is configured circulate a reaction solution (7) across the reaction chamber and wherein the extraction section is configured to circulate an extraction solution (8) across the extraction chamber.

In an embodiment of the invention, the polarity of the reaction solution is such that the membrane (4) separating the reaction chamber from the extraction chamber, is saturated with the reaction solution. Expressed differently, the membrane surface polarity matches with the polarity of the reaction solution, such that the membrane is saturated with the later. The polarity of the reaction solution is primarily dependent of the reaction solvent being used. Consequently, in a particular embodiment the polarity of the reaction solvent is such that the membrane (4) separating the reaction chamber from the extraction chamber, is saturated with the reaction solvent.

In an embodiment of the invention, the reaction solution is based on a polar aprotic reaction solvent comprising and any kind of coupling reagent as well as FMOC deprotection system known to the person skilled in the art of peptide synthesis, wherein the reaction solvent is in particular selected from ethyl acetate, anisole or DMF; more in particular anisole.

For the extraction solution (8) it has been observed that best results in circulating the reaction solution and the extraction solution are achieved when the extraction solution is a reactive extraction solution, in that it reacts with the reagents and/or by-products crossing the membrane. The reactivity of the extraction solution is primarily dependent of the extraction solvent being used. Consequently, in a particular embodiment the extraction solvent is a reactive extraction solvent, in that it reacts with the reagents and/or by-products crossing the membrane. If for example an amine is to be removed, the extraction solution is reactive when it is an acidic extraction solution. Being acidic it would protonate the extracted amine and prevent it from reverse crossing the membrane, i.e. from the extraction chamber into the reaction chamber. Though reactive extraction is the most efficient, it is not obliged. A difference in solubility between the feed phase and the extraction phase can also be used, though this is typically less efficient than reactive extraction.

In an embodiment of the invention, the extraction solvent is selected from an aqueous acid solution or ethanol. Where an aqueous acid solution, such as aqueous sulphuric acid is being preferred.

### Brief description of the figures

Various aspects of the invention will now be discussed in greater detail with reference to the appended drawings, with the same reference numerical illustrating the same attributes.
Figure 1 - Schematic representation of the separation challenge in peptide synthesis.
Figure 2 - Schematic representation of a liquid phase peptide synthesis system according to the invention
Figure 3 - Ln (1-extraction efficiency) over time of the TMB-ester (full lines) in comparison with the DBF-deta adduct (dotted lines) and the DBF-piperidine adduct (squares) over the Puramem-selective membrane in Anisole.

### Description of embodiments

### Cross reference numbers

1. Housing
2. Reaction Chamber
3. Extraction Chamber
4. Membrane
5. Feed Section
6. Extraction Section
7. Reaction Mixture, also referred to as Reaction Solution
8. Extraction Mixture, also referred to as Extraction Solution
9. Reaction Supply Container
10. Reaction Chamber Inlet
11. Reaction Chamber Outlet
12. Filling Opening
13. Extraction Supply Container
14. Extraction Chamber Outlet
15. Extraction Chamber Inlet
16. Reaction Solution Supply Means
17. Reaction Solution Pump
18. Reaction Solution Feed Line
19. Extraction Solution Supply Means
20. Extraction Solution Pump
21. Extraction Solution Feed Line

Peptides are important biomolecules. As mentioned hereinbefore, it is an object of the present invention to provide improved peptide synthesis techniques to cost-effectively achieve quality and yield of peptides while reducing batch variability.

To said extend the present inventors have discovered that the use of DETA as base reagent addresses the separation challenge current peptide synthesis methods are faced with. Its use is particularly beneficial in LPPS and greatly reduces the amount of extraction solution to achieve purity specifications comparable to solid phase tide synthesis, but requiring large volumes of solvents to wash away impurities from the reaction product.

With reference to Figure 1, stepwise peptide synthesis includes in each cycle a step wherein the amino protective group needs to be removed in order to have the amino acid in the growing peptide available for the next coupling reaction with an N-terminal protected amino acid (see also Scheme 1 in the Examples hereinafter). This deprotection is typically being done using piperidine as base reagent with the formation of a so called DBF-base adduct. Prior to the next coupling reaction;
- Excess base needs to be removed to prevent unwanted side reactions leading to sequence errors.
- Excess deprotected amino acid needs to be removed to prevent sequence errors.
- Free DBF needs to be removed as it can polymerise in-situ or add to the peptide N-terminal leading to non-removable N-capping; and
- the presence of DBF-base adduct is an unwanted reaction ballast that can affect downstream processing.

In liquid phase peptide synthesis system that are based on the presence of a housing (1) comprising a reaction chamber (2) and an extraction chamber (3) separated from each other by a membrane (4), by which the aforementioned products are removed from the reaction chamber. In existing systems, such as for example described in US patent US 6,670,173 the membrane is either a semi-permeable membrane that allows a bi-directional exchange of products and liquids between the chambers, with a selectivity that is based on size exclusion; or a membrane is used that acts as a solid phase extraction membrane with an affinity for the reagents and by-products present in the reaction chamber.. With the semi-permeable membranes, transport across the membrane is primarily driven by a concentration gradient for reagents and/or by-products between the reaction solution and the extraction solution, whereas with the solid phase extraction membrane, the membrane will be saturated with the reaction solution and pull the excess reagents and by-products from the reaction chamber across the membrane.

The present invention is based on the finding that using DETA as the base reagent in the N-terminal deprotection step yields a quasi-complete conversion of the Fmoc protecting group into the corresponding DBF-base adduct, thus reducing the number of compounds to be removed from the reaction chamber. In addition, when used in an LPPS system comprising a solid phase extraction membrane, said DBF-base adduct is more efficiently removed from the reaction chamber. This is in particular the case when the solid phase extraction membrane is a low polarity membrane, such as the polymeric membranes selected from a silicone - coated polyacrylonitrile or a polystyrene modified hymem membrane.

Compared to piperidine, the base reagent typically used, DETA has the following benefits;
- It forms more efficiently than piperidine the DBF-base adduct compound than piperidine in the solvent used - from results generated by Unibo, they showed under the same conditions less than 5 % of DBF was present in the reaction mixture after deprotection. Whereas, with piperidine this can be as much as 15%
- Removal of the DBF-base adduct is more efficient in the membrane system used than the removal of DBF
- Deta is not a controlled substance as piperidine is which is used in drug manufacture
- Deta costs less than piperidine
- Deta is typically used in the polymer industry
- Piperidine reacts with some pump seal materials, Deta does not.

To said effect the solid phase extraction membranes used in the LPPS system are selected to have a surface polarity that matches the polarity of the reaction solution, to have an affinity for the reagents and/or by-products, and to have a polarity contrary to the polarity of the extraction solution. Doing so, the membrane will be saturated with the reaction solution and pull the excess reagents and by-products from the reaction chamber across the membrane.

In a particular embodiment the solid phase extraction membranes used are low polarity membrane, such as the polymeric membranes selected from a silicone - coated polyacrylonitrile or a polystyrene modified hymem membrane, with the silicone - coated polyacrylonitrile being most preferred.

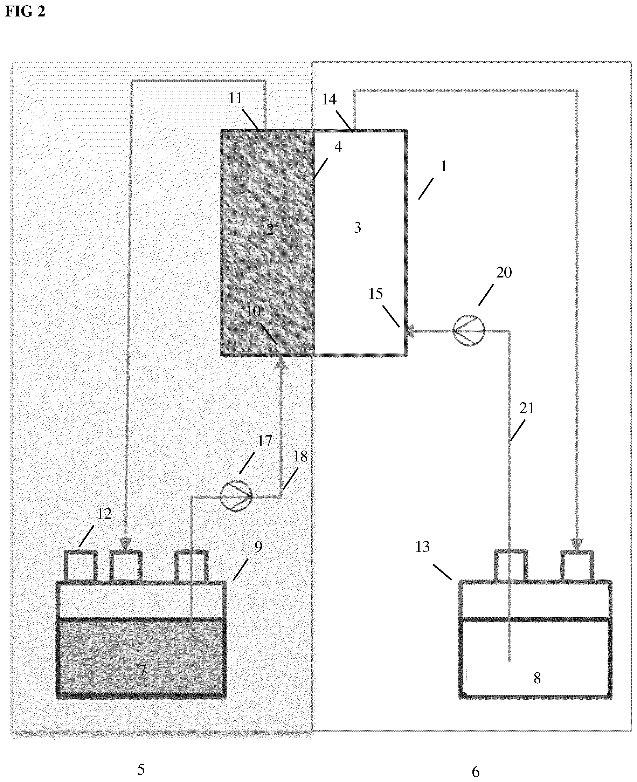

The system further comprises a feed section (5) and an extraction section (6). In the feed section a reaction mixture / solution (7) is circulated across the reaction chamber and in the extraction section an extraction mixture / solution (8) is circulated across the extraction chamber. In the embodiment shown in Figure 2, a reaction mixture is fed from a reaction supply container (9) by means of a reaction chamber inlet (10) into the reaction chamber. Via a reaction chamber outlet (11) the reaction solution is recirculated into the reaction supply container. To replenish spent reagents, the container is equipped with an additional filling opening (12).

Where the invention is based on the identification of DETA as a suitable base reagent in peptide synthesis, its application in LPPS has a further benefit when it comes to the reaction solution being used. Today, DMF is the industrial standard solvent used for peptide synthesis. Unfortunately, DMF is also classified as a hazardous solvent and it would be desirable to have it replaced by another solvent. Ethyl acetate is known as such an alternative, but has the downsize in being problematic for some pump seal materials. In the combination with DETA it has now been found that anisole can be used as a reaction solution instead of DMF. Compared to DMF, Anisole is classed as a recommended solvent for industrial use in the solvent selection guides, and it was efficient in the DETA base removal, where DMF failed. Also DMF tends to be lost into the extraction phase across the membrane.

At the extraction section (6) a similar configuration exists. An extraction solution is fed from an extraction supply container (13) into the extraction chamber. Via an extraction chamber outlet (14) the extraction solution is recirculated into the extraction supply container through an extraction chamber inlet (15). In the shown embodiment, the extraction section consists of a closed loop. As mentioned before, combining the extraction solution with a solid phase extraction membrane with affinity for the reaction solution comprising the reagents and by-products, enhances the transport of excess reagents and by-products across the membrane into the extraction solution and enables a closed loop of the extraction solution at the extraction section, in particular in said instance where the extraction solution is a reactive extraction solution.

As used herein, a reactive extraction solution, is chemical species constituting, or present in, the extraction solution that reacts with the extracted chemical species, in the instant application with at least the excess reagents and preferably also with possible by-products. Reactive liquid extraction processes are known, such as the extraction of carboxylic acids with amine extractants, or vice-versa (Tamada, J. A.; Kertes, A. S.; King, C. J. "Extraction of carboxylic acids with amine extractants. 1. equilibria and law of mass action modeling". Ind. Eng. Chem. Res. 29, pages 1319-1326, 1990).

In a particular embodiment the reactive extraction solution in an aqueous acid solution, such as HCl or sulphuric acid. In a preferred embodiment the extraction solution is an aqueous sulphuric acid solution. Unlike HCl, sulphuric acid is not corrosive to the pump and unit material, and its acidic nature can protonate all impurities containing base functionality, thus preventing any reverse diffusion back into the feed solution.

### Numbered embodiments of the invention

1. Use of diethylenetriamine (DETA) as base reagent in the synthesis of peptides.
2. The use of embodiment 1, wherein the synthesis of peptides is by means of membrane technology, like Solid Phase Peptide Synthesis (SPPS) or Liquid Phase Peptide Synthesis (LPPS).
3. The use of embodiments 1 or 2, wherein the synthesis of peptides is by means of Liquid Phase Peptide Synthesis (LPPS).
4. A method for the cleavage of a Fmoc amino acid protective group in peptides synthesis, comprising contacting the Fmoc amino acid protective group with a solution comprising DETA, thereby cleaving the Fmoc from said Fmoc protected amino acid group.
5. The method of embodiment 4, wherein the peptide synthesis is by means of membrane technology, like Solid Phase Peptide Synthesis (SPPS) or Liquid Phase Peptide Synthesis (LPPS); in particular by means of LPPS.
6. The method of embodiments 4 or 5, wherein the solution comprising DETA is a solution of DETA in a reaction solvent selected from ethyl acetaten anisole or DMF.
7. The method of embodiment 6, wherein the solution of DETA is a solution of DETA in anisole; in particular a solution from about 5 Vol *%* to 30 Vol *%* of DETA in anisole.
8. The use of embodiment 3, or the method of embodiment 5, wherein the LPPS is by means of a system having a housing (1) comprising a reaction solution (7) in a reaction chamber (2) and an extraction solution (8) in an extraction chamber (3) separated from each other by a membrane (4), characterized in that the membrane is configured to act as a solid phase extraction membrane between the reaction chamber and extraction chamber with an affinity for reagents and by-products present in the reaction chamber.
9. The use or method of embodiment 8, comprises a step of feeding a solution of DETA into the reaction chamber thereby contacting the Fmoc protected amino acid groups present within said reaction chamber with DETA, and effecting removal of Fmoc from said Fmoc protected amino acid groups.
10. The use or method of embodiment 8, wherein the membrane is a polymeric or ceramic nanofiltration membrane, in particular a ceramic nanofiltration membrane with a Molecular Weight Cut-Off (MWCO) of less than 900.
11. The use or method of embodiment 8, wherein the membrane is a polymeric membrane selected from a silicone - coated polyacrylonitrile or a polystyrene modified hymem mebrane, the silicone - coated polyacrylonitrile being most preferred.
12. The use or method according to any one of the preceding embodiments, wherein reaction solution comprises a reaction solvent selected from ethyl acetate, anisole or DMF; more in particular anisole.
13. The use or method according to any one of the preceding embodiments, wherein the extraction solution is a reactive solution, in that it reacts with the reagents and/or by-products crossing the membrane.
14. The use or method according to embodiment 13, wherein the extraction solvent is selected from an aqueous acid solution or ethanol; an aqueous acid solution, such as aqueous sulphuric acid being preferred.
15. A method for the cleavage of a Fmoc amino acid protective group in a LPPS system having a housing (1) comprising a reaction solution (7) in a reaction chamber (2) and an extraction solution (8) in an extraction chamber (3) separated from each other by a solid phase extraction membrane (4) with an affinity for reagents and by-products present in the reaction chamber; said method comprising a step of feeding a solution of DETA into the reaction chamber thereby contacting Fmoc protected amino acid groups present within said reaction chamber with DETA, and effecting removal of Fmoc from said Fmoc protected amino acid groups.
16. The method of embodiment 15, wherein;
   - the reaction solution comprises anisole as reaction solvent;
   - the membrane is membrane is a polymeric membrane selected from a silicone - coated polyacrylonitrile or a polystyrene modified hymem mebrane, the silicone - coated polyacrylonitrile being most preferred; and wherein
   - the extraction solvent is selected from an aqueous acid solution or ethanol; an aqueous acid solution, such as aqueous sulphuric acid being preferred.
17. The method of embodiments 15 or 16, wherein the solution of DETA is a solution of DETA in anisole.

### Examples

In case of peptide synthesis each chain step consists of reacting an amino acid (AAₓ) or short chain peptide with C-terminal protection (C-PG), typically trimethoxybenzyl (Tmob) schematically shown in scheme 1 below, with an amino acid (AAₓ₊₁) with a protecting group on the N-terminal of the amino acid (N-PG), typically the Fmoc protecting group, schematically shown below.

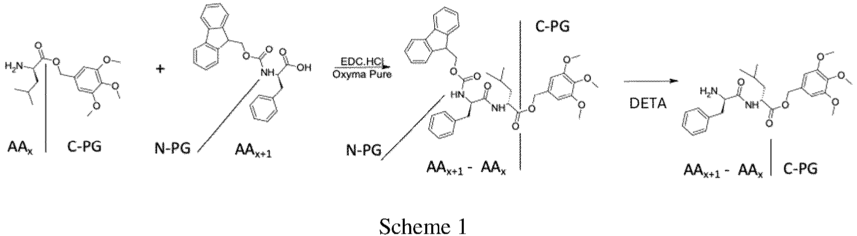

Conceptually the extraction could be carried out at either directly after chain elongation with the N-terminal protection still on the molecule, after removal of the N-terminal protection as suggested in the overall synthesis sequence above or after both. Experiments have shown the process to work best after removal of the N-terminal protection i.e. only one membrane operation per chain elongation step is then required.

### Example 1

In each of the elongation steps Piperidine, DETA or one of the bases mentioned in Table 2 is added to remove the N-terminal protecting group, yielding the AAₓ - C-PG in the above scheme as a starting product. For the subsequent synthesis it is important that the DBF-Base adduct, e.g. DBF-Piperidine or DETA adduct, see scheme 2 below, is removed from the reaction medium.

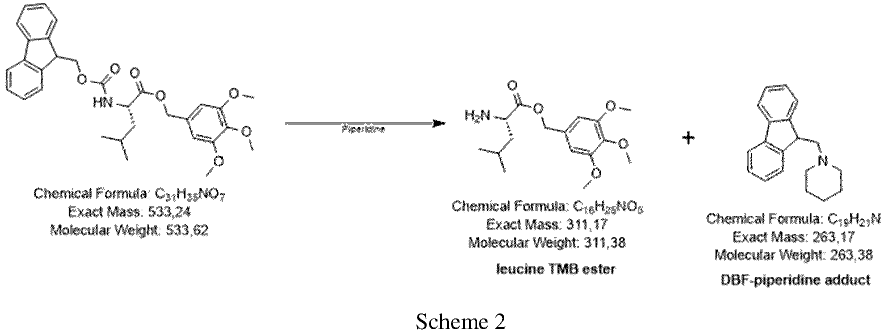

A HPLC analysis has been performed for each of the bases tested to observe the conversion rate, i.e. deprotection of the FMOC-amino acid (1) into the DBF-base adduct (2) and free DBF product (3) wherein a conversion into the DBF-base adduct is being preferred, see scheme 3 below.

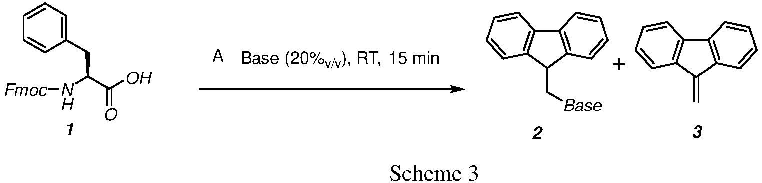

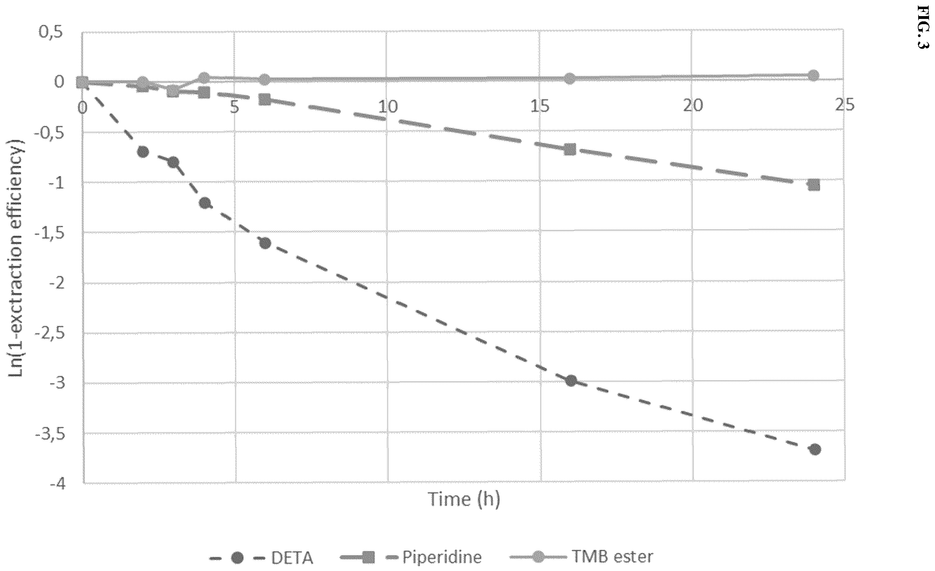

Using different membranes and feed solvents (Table 1) it has been found that extraction efficiency is working best using Anisole as solvent in the reaction solution and using DETA as base reagent in combination with reactive extraction using aqueous acid as extractant. Figure 3 compares the extraction efficiency for the DBF-DETA and DFB-Piperidine adduct over the Puramem-selective membrane. The removal of the DBF-DETA adduct is much faster when compared to the DBF-piperidine adduct.

**Results summary - Table 1:**

| membrane | Feed solvent | Duration of extraction (min) | *%* in retentate | | |
|---|---|---|---|---|---|
| | | | Leucine-TMB | piperidine | Deta |
| Pm-selective | Anisole | 400 | 95 | 25 | |
| | Anisole | 400 | 95 | / | 4 |
| | DMF | 400 | 95 | 34 | / |
| 1 nm C₁ TiO₂ | Ethyl acetate | 300 | 90 | 56 | / |
| | Ethyl acetate | 300 | 85 | / | 10 |
| 5 nm PMMA-TiO₂ | Anisole | 300 | 95 | 90 | / |
| | Anisole | 300 | 70 | / | 26 |
| Extraction solvent in all cases is aqueous H₂SO₄ at pH 2 | | | | | |

Table 1 shows that under all conditions tested, DETA outperforms piperidine as base reagent in the synthesis of peptides by means of a LPPS.

Best results are obtained for the Puramem-selective (polymeric) membrane using Anisole as Feed solvent, but even for the ceramic membranes 1nm C₁ TiO₂ and 5nm PMMA-TiO₂, the percentage of DETA retentate is always significantly lower when compared to piperidine as base reagent.

**Results summary - Table 2:**

| DETA | | Piperidine | | DEAPA | | tBuNH2 | |
|---|---|---|---|---|---|---|---|
| conversion | **2/3** | Conversion | **2/3** | Conversion | **2/3** | Conversion | **2/3** |
| 100*%* | 92 / 8 | 100*%* | 94/ 6 | 100*%* | 20 / 80 | 100*%* | 0 / 100 |

Table 2. Deprotection of the FMOC amino acid at room temperature in DMF. All deprotection were completed after 15min, analysis performed by HPLC.

Best results are obtained for piperidine and DETA, but compared to piperidine, DETA (diethylenetriamine; CAS N° - 110-40-0) as the base reagent provides the combination of two primary amines that react with the excess of the activated amino acid, while the secondary amine is promoting Fmoc deprotection trapping DBF resulting in a highly efficient removal of the DBF-base adduct from the reaction environment as evident from Table 1 above.

## Claims

1. Use of diethylenetriamine (DETA) as base reagent in the synthesis of peptides.

2. The use of claim 1, wherein the synthesis of peptides is by means of membrane technology, like Solid Phase Peptide Synthesis (SPPS) or Liquid Phase Peptide Synthesis (LPPS).

3. The use of claims 1 or 2, wherein the synthesis of peptides is by means of Liquid Phase Peptide Synthesis (LPPS).

4. A method for the cleavage of a Fmoc amino acid protective group in peptides synthesis, comprising contacting the Fmoc amino acid protective group with a solution comprising DETA, thereby cleaving the Fmoc from said Fmoc protected amino acid group.

5. The method of claim 4, wherein the peptide synthesis is by means of membrane technology, like Solid Phase Peptide Synthesis (SPPS) or Liquid Phase Peptide Synthesis (LPPS); in particular by means of LPPS.

6. The method of claims 4 or 5, wherein the solution comprising DETA is a solution of DETA in a reaction solvent selected from ethyl acetaten anisole or DMF.

7. The method of claim 6, wherein the solution of DETA is a solution of DETA in anisole; in particular a solution from about 5 Vol*%* to 30 Vol*%* of DETA in anisole.

8. The use of claim 3, or the method of claim 5, wherein the LPPS is by means of a system having a housing (1) comprising a reaction solution (7) in a reaction chamber (2) and an extraction solution (8) in an extraction chamber (3) separated from each other by a membrane (4), **characterized in that** the membrane is configured to act as a solid phase extraction membrane between the reaction chamber and extraction chamber with an affinity for reagents and by-products present in the reaction chamber.

9. The use or method of claim 8, comprises a step of feeding a solution of DETA into the reaction chamber thereby contacting the Fmoc protected amino acid groups present within said reaction chamber with DETA, and effecting removal of Fmoc from said Fmoc protected amino acid groups.

10. The use or method of claim 8, wherein the membrane is a polymeric or ceramic nanofiltration membrane, in particular a ceramic nanofiltration membrane with a Molecular Weight Cut-Off (MWCO) of less than 900.

11. The use or method of claim 8, wherein the membrane is a polymeric membrane selected from a silicone - coated polyacrylonitrile or a polystyrene modified hymem mebrane, the silicone - coated polyacrylonitrile being most preferred.

12. The use or method according to any one of the preceding claims, wherein reaction solution comprises a reaction solvent selected from ethyl acetate, anisole or DMF; more in particular anisole.

13. The use or method according to any one of the preceding claims, wherein the extraction solution is a reactive solution, in that it reacts with the reagents and/or by-products crossing the membrane.

14. The use or method according to claim 13, wherein the extraction solvent is selected from an aqueous acid solution or ethanol; an aqueous acid solution, such as aqueous sulphuric acid being preferred.

15. A method for the cleavage of a Fmoc amino acid protective group in a LPPS system having a housing (1) comprising a reaction solution (7) in a reaction chamber (2) and an extraction solution (8) in an extraction chamber (3) separated from each other by a solid phase extraction membrane (4) with an affinity for reagents and by-products present in the reaction chamber; said method comprising a step of feeding a solution of DETA into the reaction chamber thereby contacting Fmoc protected amino acid groups present within said reaction chamber with DETA, and effecting removal of Fmoc from said Fmoc protected amino acid groups.

16. The method of claim 15, wherein;
- the reaction solution comprises anisole as reaction solvent;
- the membrane is membrane is a polymeric membrane selected from a silicone - coated polyacrylonitrile or a polystyrene modified hymem mebrane, the silicone - coated polyacrylonitrile being most preferred; and wherein
- the extraction solvent is selected from an aqueous acid solution or ethanol; an aqueous acid solution, such as aqueous sulphuric acid being preferred.

17. The method of claims 15 or 16, wherein the solution of DETA is a solution of DETA in anisole.
